Europäisches Patentamt

European Patent Office

Office européen des brevets

(19)

(11) Publication number: **0 352 717**
**A2**

# (12) EUROPEAN PATENT APPLICATION

(21) Application number: **89113638.4**

(51) Int. Cl.4: **C12Q 1/58 , C12M 1/40**

(22) Date of filing: **25.07.89**

(30) Priority: **25.07.88 US 224055**
**27.04.89 US 344140**

(43) Date of publication of application:
**31.01.90 Bulletin 90/05**

(84) Designated Contracting States:
**AT BE CH DE ES FR GB GR IT LI LU NL SE**

(71) Applicant: **NOVA BIOMEDICAL CORPORATION**
**200 Prospect Street**
**Waltham Massachusetts, 02254-9141(US)**

(72) Inventor: **Young, Chung Chang**
**145 Buckskin Drive**
**Weston MA 02193(US)**
Inventor: **Demko, Paul R.**
**43 Scotch Pine Road**
**Weston MA 02193(US)**
Inventor: **Winarta, Handani**
**68 Bruce Road**
**Waltham MA 02154(US)**
Inventor: **Malenfant, Arthur L.**
**75 Henry Street**
**Cambridge MA 02193(US)**
Inventor: **Welch, Robin F.**
**21 Rollins Avenue**
**Lynn MA 01901(US)**

(74) Representative: **Heidrich, Udo, Dr. jur.,**
**Dipl.-Phys.**
**Rechtsanwalt & Patentanwalt Dipl.-Phys. Dr.**
**jur. U. HEIDRICH Franziskanerstrasse 30**
**D-8000 München 80(DE)**

(54) Method, analyzer and sensor for measuring urea concentration.

(57) A method of measuring the concentration of urea in a liquid sample employing a urea sensor including urease associated with an electrode adapted to generate output response to ammonium ions. The method includes contacting the sensor with the liquid sample so that at least a portion of the urea in the liquid sample is converted by the urease to ammonium ions, the ammonium ions contacting the electrode to generate output related to the urea concentration but being unadjusted for the variation in the proportion of urea converted to ammonium ion by the urease at different urea concentrations; and adjusting the output for the variation in the proportion of urea converted to ammonium ions by the urease at different urea concentrations.

## BUN SENSOR

### Background of the Invention

The invention relates to measuring the urea concentrations of liquid samples.

Urea sensors having a urease layer and an ammonium ion sensitive electrode are known (see e.g., Niiyama et al., European Patent Application No. 84113477.8). When such sensors are contacted with a liquid sample, the urease converts urea in the sample to ammonia, which in solution forms ammonium ions. The ammonium ions contact the electrode to produce a mV output proportional to the concentration of ammonium ions.

### Summary of the Invention

In general the invention features, in one aspect, a method of obtaining an accurate measurement of the concentration of urea in a liquid sample such as whole blood. According to the method, the liquid sample is contacted with a urea sensor that includes a urease layer associated with an electrode adapted to generate output in response to ammonium ions. The urease layer converts a portion of the urea in the sample to ammonium ions, and the ions contact the electrode to generate output related to the urea concentration in the sample but being unadjusted for the variation in the proportion of urea converted to ammonium ions by the urease layer at different urea concentrations. The output is then adjusted for this variation to provide an accurate measurement of the urea concentration.

In some preferred embodiments, the method further includes obtaining a urea conversion correction slope that takes into account the variation in the portion of the urea in a sample converted to ammonium ion at different urea concentrations, and adjusting the output in accordance with the slope.

In other preferred embodiments, the output also is adjusted for the potassium and sodium ions in the liquid samples. Taking these ions into account ensures a more accurate measurement of the ammonium ion concentration.

The method can be carried out using an analyzer for measuring the urea concentration of a liquid sample. The analyzer includes the urea sensor and a microprocessor adapted to receive the output from the electrode and to adjust the output for the variation in the proportion of urea converted to ammonium ions by the urease at different urea concentrations.

In some preferred embodiments, the analyzer further includes a data output port connected to the microprocessor to provide the measurement to the operator of the analyzer.

In other preferred embodiments, the microprocessor is further adapted to obtain a urea conversion correction slope that takes into account the variation in the portion of the urea in a sample converted to ammonium ion at different urea concentrations.

The proportion of urea converted to ammonium ions by a given urease layer can vary for different urea concentrations. There is, however, a linear relationship between the proportion of urea converted and the concentration of urea in the sample. The above invention uses this linear relationship to provide accurate measurments of urea concentrations in test samples.

Another aspect of the invention features a urea sensor that includes an ammonium-ion sensitive electrode and an adjacent 1 to 50 microns (preferably 4 to 10 microns) thick urease layer. The urease layer includes a polymeric (e.g., polyester) membrane having a pore size of 0.1 to 10 microns (preferably 0.8 to 2 microns), and urease immobilized on the polymeric membrane. Preferably the urease is immobolized on the face of the membrane adjacent the ammonium ion electrode, but not on the membrane's liquid sample contacting face. The sensor is durable, provides consistent results, and has a good response time. The limited pore size does not allow the passage of large interfering substances, and meters the passage of urea into the membrane. Moreover, in the preferred embodiment, not having urease on the surface contacting face of the membrane protects the enzyme from being contaminated by extraneous large liquid sample components.

Other features and advantages of the invention will be apparent from the following description of the preferred embodiments thereof, and from the claims.

## Description of the Preferred Embodiment

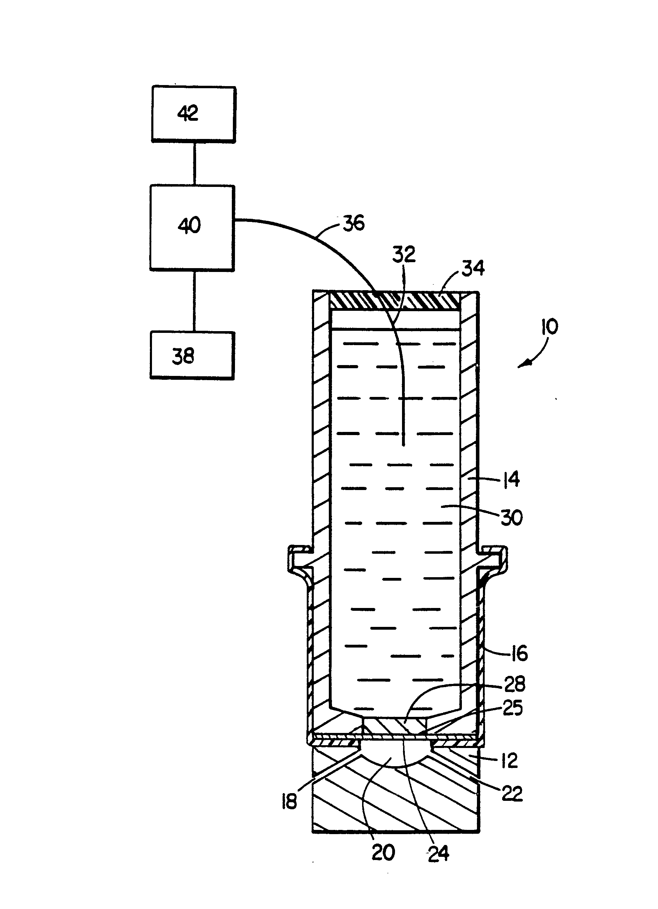

The drawing is first described.

### Drawing

The Figure is a sectional view of a urea analyzer of the invention.

Referring to the Figure, urea sensor 10 includes flow cell 12, electrode body 14, and urease membrane cap 16.

Flow cell 12 is constructed of a suitable liquid impervious material (e.g., polystyrene, polymethacrylate) and has liquid inlet 18, chamber 20, and liquid outlet 22.

Electrode body 14 (O.D., e.g., 0.33"; I.D., e.g., 0.21"; height, e.g., 1.41") includes ammonium ion sensitive electrode 28 (e.g., a standard nonactin-based ammonium ion selective membrane electrode). Electrode body 14 further includes chamber 30, which is filled with a suitable reference solution; internal reference electrode 32 (e.g., Ag/AgCl wire); and a silicone plug 34. The walls of electrode body 14 can be made of any suitable material (e.g., PVC).

Urease membrane cap 16 (made of, e.g., delrin) fits over the sample contacting end of electrode body 14 and includes a urease layer 24. Urease layer 24 consists of a 4 to 10 microns thick (more preferably 5 microns thick) polyester membrane having a pore size of 0.1 to 10 microns (more preferably 0.8 to 2.0 micron most preferably 0.8 micron). The urease is immobolized only on inner face 25 of the membrane, i.e., the face adjacent to electrode 28. The urease is immobolized on the polyester membrane by conventional methods, such as gluteraldehyde crosslinking.

Having the urease immobolized only on inner face 25 prevents direct exposure of the enzyme to the bulk sample solution, as only small molecules such as urea itself can diffuse throughout the pores. Accordingly, large substances such as red blood and proteins are prevented from contacting the urease, increasing the functional lifetime of the urease layer.

The thinness of the polyester membrane results in the sensor having a fast response time. Moreover, the thinness provides the sensor with an excellent washout time.

The limited pore size, in addition to not allowing the ready passage of large substances through the membrane, meters the passage of urea through the membrane and into contact with the urease. This helps prevent the enzyme from being saturated with urea, and leads to a consistent sensor response.

Lead wire 36 connects the sensor to an amplifier, which in turn is connected to computer 40, e.g., an Intel SBC 80/10B computer including a 8080A CPU microprocessor. Data output port 42, associated with computer 40, provides data in readable form to the operator. A standard external reference electrode 38 also is connected to computer 40.

In use, a liquid sample (e.g., an undiluted clinical sample such as serum or whole blood) flows into chamber 20 through liquid inlet 18. Urea in the liquid sample contacts the urease in urease layer 24, and a portion of the urea is converted to ammonia, which forms ammonium ions in solution. The ammonium ions generated contact electrode 28 to produce an electrical signal (or output). The signal is passed to computer 40 via lead wire 36, and is proportional to the concentration of ammonium ion in the solution, and thus proportional to the portion of urea in the liquid sample that has been converted to ammonia. The microprocessor is programmed to do the relevant calculations, and the results are transmitted in readable form to the operator through output port 42.

If urease layer 24 converted the same proportion of urea to ammonium ions at all urea concentrations (and ignoring for the moment the effect of $K^+$ and $Na^+$ on the measurement), the urea concentration of an unknown sample could be determined by measuring the mV output of sensor 10 for two standards containing known urea concentrations; determining the Nerstian slope relating mV to ammonium ion concentration; obtaining the mV output of a test sample; and using the Nernst equation to calculate the urea concentration of the test sample. The % conversion of urea by urease layer 24 varies, however, with the urea concentration in the liquid sample.

For a given urease layer, there is a linear relationship between the urea concentration and the % conversion. The method of the invention takes this relationship into account to provide accurate measurements of urea concentrations in test samples.

The most preferred method also takes into account the output from ammonium ion sensitive electrode 28 attributable to $K^+$ and $Na^+$ in the test sample.

The most preferred method includes the following four steps:

(1) Calibrating urea sensor 10 for ammonium ion using two standards;

(2) Calibrating urea sensor 10 for urea using two urea standards;

(3) Obtaining a urea conversion correction slope that takes into account the variation in the portion of the urea in a sample converted by a particular urease layer to ammonium ion at different urea concentrations; and

(4) Obtaining an accurate measurement of the urea concentration in the test sample, using the information derived during steps (1), (2), and (3). The four steps are discussed in more depth below.

In step (1), two standards (A, B) that contain known concentrations of $NH_4^+$ ($C_A$, $C_B$), $K^+$ ($C_K'$), and $Na^+$ ($C_{Na}'$) are used to determine the ammonium Nernstian slope (S) for sensor 10; $Na^+$ and $K^+$ standards are run to account for the limited sensitivity of ammonium ion electrode 28 to $Na^+$ (estimated as 350:1) and $K^+$ (estimated as 7:1). The mV output obtained with standards A and B can be expressed in conventional Nernstian form (equations (1) and (2), respectively).

$$E_A = E_{STD} + Slog(C_A + \frac{C_{Na}'}{350} + \frac{C_K'}{7}) \qquad (1)$$

$$E_B = E_{STD} + Slog(C_B + \frac{C_{Na}'}{350} + \frac{C_K'}{7}) \qquad (2)$$

Equation (1) can be subtracted from equation (2) to give equation (3).

$$\Delta E_1 = E_B - E_A = Slog \frac{C_B + \frac{C_{Na}'}{350} + \frac{C_K'}{7}}{C_A + \frac{C_{Na}'}{350} + \frac{C_K'}{7}} \qquad (3)$$

In equation (3), the only unknown is slope S, which can be readily solved because $E_A$ and $E_B$ (the mV output measured for the standards) and the various concentrations are known.

In step (2), the urea conversion factors ($f_C$ and $f_D$)--the fraction (or portion) of urea actually converted to ammonium ions at a specific urea concentration--are calculated by using two standards (C, D) having known concentrations of urea ($C_C$, $C_D$), sodium ion ($C_{Na}$ for sample C; $C_{Na}''$ for sample D), and potassium ions ($C_K$ for sample C; $C_K''$ for sample D). The mV outputs obtained with standards C and D can be expressed in conventional Nernstian form (equations (4) and (5), respectively).

$$E_C = E_{STD} + Slog[(C_C F_C) + \frac{C_{Na}}{350} + \frac{C_K}{7}] \qquad (4)$$

$$E_D = E_{STD} + Slog[(C_D f_D) + \frac{C_{Na}''}{350} + \frac{C_K''}{7}] \qquad (5)$$

By subtracting equation (1) from equation (4), equation (6) is obtained.

$$\Delta E_2 = E_C - E_A = Slog \frac{C_C f_C + \frac{C_{Na}}{350} + \frac{C_K}{7}}{C_A + \frac{C_{Na}'}{350} + \frac{C_K'}{7}} \qquad (6)$$

By subtracting equation (2) from equation (5), equation (7) is obtained.

4

$$\Delta E_3 = E_D - E_B = S \log \frac{C_D f_D + \dfrac{CNa''}{350} + \dfrac{Ck''}{7}}{C_B + \dfrac{CNa'}{350} + \dfrac{Ck'}{7}} \quad (7)$$

$E_A$, $E_C$, and $E_D$; $C_C$ and $C_D$; $C_{Na}'$, $C_{Na}''$, and $C_{Na}$; $C_K'$, $C_K''$, and $C_K$; and S are known quantities. Accordingly, $f_C$ and $f_D$ can be determined from equations (6) and (7), respectively.

The linear relationship between the concentration of urea and the % conversion of urea by a given urease layer allows the values obtained in equations (6) and (7) to be expressed in conventional y = mx + b terms (equations (8) and (9) respectively).

$\log C_C f_C = m\log C_C + b$ (8) $\log C_D f_D = m\log C_D + b$ (8) In step (3), the urea conversion correction slope m and constant b are obtained by solving for the values in equations (8) and (9).

In step (4), an accurate measurement of the urea concentration in a test sample (X) is determined. Initially, the Na$^+$ and K$^+$ concentrations ($C_{Na_x}$, $C_{K_x}$) of the test samples are obtained using standard potassium and sodium sensors. Next, the $C_X f_X$ value for the test sample (where $C_X$ is the urea concentration of the test sample and $f_X$ is the pertinent urea conversion factor) is determined by subtracting conventional Nernstian equation (10) from equation (4), to give equation (11), and substituting in the known values for $E_X$ (mV output from the test sample), $E_C$, S, $C_C$, $f_C$, $C_{Na_x}$, $C_{K_x}$, $C_{Na}$, and $C_K$.

$$E_x = E_{STD} + S\log\left[(C_x f_x) + \frac{C_{Na_x}}{350} + \frac{C_{K_x}}{7}\right] \quad (10)$$

$$\Delta E_4 = E_x - E_c = S\log \frac{C_x f_x + \dfrac{C_{Na_x}}{350} + \dfrac{C_{K_x}}{7}}{C_c f_c + \dfrac{C_{Na}}{350} + \dfrac{C_K}{7}} \quad (11)$$

Finally, the urea concentration of the sample, $C_X$, is determined using equation (12) (m and b for the urease layer having been determined in step (3)):

$\log C_X f_X = m\log C_X + b$ (12)

For the purposes of this application, $E_X$ is the output related to the urea concentration of the test sample that is unadjusted for variation in the proportion of urea converted to ammonium ion at different urea concentrations. The steps of solving for $C_X f_X$ and then determining the value of $C_X$ using equation (12) are the preferred approach to adjusting this output for this concentration variation to provide an accurate measure of the urea.

The urea concentrations of other test samples can be determined using sensor 10 simply by repeating step (4).

A suitable program in assembly language for performing the above calculations on an 8080A CPU microprocessor is included as an appendix to this application.

Other embodiments are within the following claims.

APPENDIX

```
'S3( *   MBUSMA          -MATH FOR CALCULATING BUN mM, PART 1)
!
! --- MATH FOR CALCULATING BUN mM
!
! BINARY CONCENTRATION RETURNED IN BINBU IS
!      ALWAYS IN mM. ROUNDED BCD CONCEN RET
!      IN CALCR IS IN mM IF <RSET>=0. IN mg
!      IF <RSET>=1.
!

MBUSMA:           PUBLIC    MBUSMA

                  AACM      CXROA
                  RCLM      BUDST         !get sample mV's
                  MINM      AMVBU         !EaBU-EnBU
                  DIVM      SLOPNH        !divide by NH4 slope
                  EXPM      CXROA         !take the exponential
                  AACM      CALCR
                  RCLM      NHCABU        !get STD-A conversion
                  MULM      BUMUL_RO      !multiply by STD-A constant
                  PLSM      NHCNA_RO      !add STD-A constant
                  MULM      CXROA         !multiply
                  AACM      CXROA
                  RCLM      BINK          !get K concentration
                  DIVM      KCORR_RO      !divide by K correction factor
                  AACM      CALCR
                  MINM      CXROA         !subtract K contribution
                  AACM      CXROA
                  RCLM      BINNA         !get Na concentration
                  DIVM      NACORR_RO     !divide by Na correction factor
                  AACM      CALCR
                  MINM      CXROA         !subtract Na contribution
                  STRM      BINBU         !store for future calculations
                  MENDM
!
! SECOND STEP IN BUN CALCULATIONS
!
                  PUBLIC    MBUSMB
MBUSMB            PROC

                  AACM      CALCR
                  RCLM      BINBU         !get BUN current calculation
                  IFPM      ..10          !If pos., do not indicate error
                  BRM       ..calc_error  !Indicate error
..10
                  IFNEGM    ..20          !Don't take log of neg number
                  LOGM      CALCR         !take natural log
..20
                  STRM      BINBU         !store for future calculations
                  MENDM

..calc_error
                  ! Place error to indicate that neg log
                  ! would have been required.
                  MVI       B,ERBUCC
                  CALL      ERMGR
```

```
                RET

                ENDPROC

    ;
    ; FINAL STEP IN BUN CALCULATIONS
    ;
    ;
                PUBLIC    MBUSMC
MBUSMC                  PROC
                AACM      CALCR
                RCLM      BINBU           ;get BUN current calculation
                MINM      NHCINT          ;subtract intercept calculation
                DIVM      SLPNHC          ;divide by NH4 slope
                EXPM      CALCR           ;take the exponential
                PLSM      BUADD_RD
                DIVM      BUDIV_RD
                DIVM      BUOFD           ;divide by BUN offset
                STRM      BINBU           ;store for future calculations
                MULM      MOBMUL_RD,FSET     ;multiply by mO% if called for
                PLSM      BURFF_RD,FSET      ;add round-off factor
                BCDM      CALCR           ;convert for display
                AACM      DACCA
                MINM      EZBU            ;chnck E-Zero range
                IFPM      ..10
                CHSM      DACCA
    ..10
                MINM      EZTLBU_RD       ;CHK E-ZERO DRIFT
                IFNEGM    ..20
                SRM       ..BUN_EZER      ;if drift call error routine
    ..20
                AACM      CKRGA
                RCLM      BINBU           ;get concentration
                MINM      BUMAX_RD        ;check for out of range error
                IFNEGM    ..30
                SRM       ..BUN_TOO_HIGH
    ..30
                AACM      CKRGA
                RCLM      BINBU
                MINM      BUMIN_RD
                IFPM      ..40
                SRM       ..BUN_TOO_LOW   ;if out of range call error routine
    ..40
                MENDM

    ..BUN_EZDR      ;subroutine to place
                    ;E-ZERO drift error code
                MVI       B,EREZDU
                CALL      ERMGR
                RET

    ..BUN_TOO_HIGH  ;subroutine to place error
                    ;code for over range
                MVI       B,ERBURH
                CALL      ERMGR
                RET


    ..BUN_TOO_LOW   ;subroutine to place error
                    ;code for under range
                MVI       B,ERBURL
                CALL      ERMGR
                RET

                ENDPROC
```

Claims

7

1. A method of measuring the concentration of urea in a liquid sample employing a urea sensor including urease associated with an electrode adapted to generate output in response to ammonium ions, said method characterized by the steps of:

contacting said sensor with said liquid sample so that at least a portion of the urea in said liquid sample is converted by said urease to ammonium ions, said ammonium ions contacting said electrode to generate output related to said urea concentration but being unadjusted for the variation in the proportion of urea converted to ammonium ion by said urease at different urea concentrations; and

adjusting said output for the variation in the proportion of urea converted to ammonium ions by said urease at different urea concentrations.

2. The method of claim 1, further characterized by obtaining a urea conversion correction slope that takes into account the variation in the portion of the urea in a sample converted to ammonium ion at different urea concentrations, and adjusting said output in accordance with said slope.

3. The method of either of the preceding claims further characterized in that said liquid sample comprises a clinical sample.

4. The method of claim 3 further characterized in that said clinical sample is serum.

5. The method of claim 3 further characterized in that said clinical sample is whole blood.

6. The method of claim 5 further characterized in that said whole blood is undiluted.

7. The method of any of the preceding claims, further characterized by adjusting said output for the potassium ion in said liquid sample.

8. The method of claim 7, further characterized by determining the potassium ion concentration of said liquid sample, and adjusting said output for the potassium ion in said liquid sample using said potassium ion concentration.

9. The method of any of the preceding claims, further characterized by adjusting said output for the sodium ion in said liquid sample.

10. The method of claim 9, further characterized by determining the sodium ion concentration of said liquid sample, and adjusting said output for the sodium ion in said liquid sample using said sodium ion concentration.

11. An analyzer for measuring the urea concentration of a liquid sample, characterized in that said analyzer comprises:

a urea sensor comprising a urease layer that converts at least a portion of the urea in a liquid sample to ammonium ions, and an electrode associated with said urease layer and adapted to generate output in response to said ammonium ions; and

a microprocessor adapted to receive said output and to adjust said output for the variation in the proportion of urea converted to ammonium ions by said urease at different ammonium concentrations to provide a measure of said urea concentration.

12. The analyzer of claim 11, further characterized in that a data outlet port is connected to said microprocessor to provide said measure to an operator of the analyzer.

13. The analyzer of claim 12, further characterized in that said microprocessor is adapted to obtain a urea conversion correction slope that takes into account the variation in the portion of the urea in a sample converted to ammonium ion at different urea concentrations.

14. A sensor for measuring the concentration of urea in a liquid sample, characterized in that said sensor comprises

an ammonium ion sensitive electrode; and

a 1 to 50 microns thick urease layer having a first face that contacts said liquid sample to allow urea in said liquid sample to pass into said urease layer and a second face positioned adjacent said ammonium ion selective electrode, said urease layer comprising a polymeric membrane having a pore size of 0.1 to 1.0 micron, and urease immobolized on said polymeric membrane.

15. The sensor of claim 14 further characterized in that said polymeric membrane comprises polyester.

16. The sensor of either of claims 14 or 15 further characterized in that said urease layer is 4 to 10 microns thick.

17. The sensor of any of claims 14-16, inclusive, further characterized in that said polymeric membrane has a pore size of 0.8 to 2.0 microns.

18. The sensor of any of claims 14-17, inclusive, further characterized in that said urease is immobolized on said second face, but not said first face, of said polymeric membrane.